# DEMANDE DE BREVET EUROPEEN

(11) **EP 2 210 569 A1**
(43) Date de publication de la demande: **28.07.2010**
(21) Numéro de dépôt: 10157477.0
(22) Date de dépôt: 27.06.2007
(51) Int. Cl.: A61C 8/00

(54) **Structure pour former de l'os**

(62) Demande divisionnaire de: 07763799.9
(71) Demandeur: Dricot, Roland, 1703 Dilbeek (BE)
(72) Inventeur: Dricot, Roland, 1703 Dilbeek (BE)
(74) Mandataire: Bird, Ariane

(57) **Abrégé**

Structure pour favoriser la formation d'os comprenant un corps comprenant essentiellement du sulfate de calcium de qualité pharmaceutique et un biomatériau phosphocalcique ou apatite sous forme de granulats poreux présentant une granulométrie moyenne en poids comprise entre 250 et 3000 µm, lesdits granulats de biomatériau présentant en tant que telles une surface spécifique comprise entre 10 et 50m² / g, une macroporosité formée de pores de diamètre moyen (moyenne en volume poreux) compris entre 2 µm et 100 µm. La structure comprend un rapport en poids sulfate de calcium dihydrate/granulats poreux phosphocalciques ou apatité étant compris entre 1:3 et 3:1.

## Description

La présente invention a pour objet une structure apte à favoriser la formation d'un os autour d'un implant, ce dernier comprenant avantageusement un corps, avantageusement en titane, présentant une portion avec un pas de vis adapté pour coopérer avec une structure osseuse de manière à assurer un premier maintien de l'implant par rapport à l'os.

On connaît par le document WO2006/089380 un implant comprenant un revêtement ostéogène comprenant une matrice ou un liant et des particules de sulfate de calcium. Le revêtement présente une microporosité formée de pores ou canaux de diamètre compris entre 20 et 70 µm. Le revêtement se présente sous la forme d'un gel et peut comprendre de l'hydroxyapatite.

On a remarqué maintenant qu'en utilisant une structure indépendante de l'implant et présentant une cavité de forme particulière, il était possible d'assurer une excellente et rapide ostéointégration, en particulier dans le maxillaire inférieur ou supérieur.

En utilisant une structure particulière présentant une cavité particulière pour recevoir une partie de l'implant, il est possible d'avoir un bon contrôle de la qualité de la structure, à la fois côté extérieur et côté intérieur (face de la cavité). Le praticien devant utiliser la structure, peut donc contrôler la qualité de la structure ainsi que celle de l'implant de manière distincte. De plus, l'implant doit souvent subir des traitements de stérilisation qui ne conviennent pas ou qui peuvent endommager la structure favorisant la formation d'os.

De plus, en usinant la cavité (mécaniquement, par exemple avec un foret ou des forets), on obtient une face intérieure présentant des pores bien adaptés à la colonisation de cellules osseuses.

La structure selon l'invention est une structure pour favoriser la formation d'os autour d'un implant avantageusement en titane, ladite structure étant adaptée pour être insérée au moins partiellement dans un os ou une structure osseuse vivante ou pour être en contact ou à proximité d'un os ou d'une structure osseuse vivante, ladite structure comprenant un corps ostéogène en une matière reconstituante d'os comprenant essentiellement du sulfate de calcium de qualité pharmaceutique et un biomatériau lentement ou non résorbable phosphocalcique ou apatite sous forme de granulats poreux présentant une granulométrie moyenne en poids comprise entre 250 et 3000µm, avantageusement comprise entre 300µm et 1000µm, lesdits granulats de biomatériau présentant en tant que telles une surface spécifique comprise entre 10 et 50m²/g, une macroporosité formée de pores ou d'alvéoles en surface de diamètre moyen compris entre 2µm et 100µm, avantageusement entre 20µm et 100µm, de préférence entre 50µm et 100 µm, ledit corps ostéogène présentant une extrémité sensiblement plane avec une ouverture prolongée par une cavité centrale avec un axe central et adaptée pour recevoir au moins une extrémité d'un implant, ladite cavité présentant au moins deux parties successives sensiblement cylindriques, à savoir au moins une première partie sensiblement cylindrique avec un premier diamètre adjacente de l'ouverture et une deuxième partie sensiblement cylindrique avec un deuxième diamètre plus petit que ledit premier diamètre, lesdites parties sensiblement cylindriques successives étant reliées entre elles par une zone intermédiaire formant un palier ou une marche s'étendant entre une partie sensiblement circulaire d'un bord d'une première partie sensiblement cylindrique et une partie sensiblement circulaire d'un bord de la deuxième partie sensiblement cylindrique, lesdites parties sensiblement circulaires desdits bords desdites parties sensiblement cylindriques étant distantes l'une de l'autre d'une distance mesurée perpendiculairement à l'axe central de la cavité comprise entre 0,03 fois et 0,25 fois le diamètre de la première partie sensiblement cylindrique, le rapport en poids sulfate de calcium calculé sous forme dihydrate /granulats poreux phosphocalcique ou apatite sous forme sèche étant compris entre 1:3 et 3:1.

De façon avantageuse, le biomatériau sous forme sèche comprend, de préférence est constitué à plus de 50% en poids (plus spécifiquement à plus de 75% en poids, par exemple plus de 90% en poids) d'agglomérats de fluoro hydroxyapatite.

Selon une forme de réalisation, le biomatériau comprend du carbonate de calcium et de l'hydroxyapatite ou de l'hydroxyapatite carbonate, le rapport en poids carbonate de calcium/hydroxyapatite du biomatériau étant compris entre 1,5 : 100 et 5 : 100, avantageusement entre 2:100 et 3,5 : 100.

Selon une forme préférentielle, le biomatériau comprend une phase organique, la phase organique représentant entre 1% en poids et 5% en poids du biomatériau sous forme sèche. Cette phase organique est avantageusement constituée de collagène et/ou de graisses et/ou de protéine.

Le biomatériau est de préférence une hydroxyapatite dérivé d'algues. Le biomatériau est par exemple préparé par la conversion hydrothermique du carbonate de calcium d'une algue en présence de phosphate d'ammonium à haute température (plus de 500°C, par exemple 600°C, 700°C, 800°C). Le procédé est conduit pour obtenir des granulats, agglomération de particules élémentaires, granulats présentant une bonne porosité microscopique et macroscopique. Par pores macroscopiques, on entend également dans le présent mémoire des cavités ou aspérités ou alvéoles formées en surface. Les alvéoles ou pores sont avantageusement recouverts au moins partiellement de micro boursouflures favorisant l'accrochage des cellules osseuses et la colonisation de la structure.

Selon une particularité, la structure présente une partie extrême dans laquelle s'étend le fond de la cavité. Cette partie extrême est avantageusement bombée.

Selon une forme de réalisation, la cavité comporte au moins trois parties sensiblement cylindriques successives à partir de l'ouverture, à savoir une première partie sensiblement cylindrique adjacente de l'ouverture présentant un premier diamètre, une deuxième partie sensiblement cylindrique adjacente de la première partie sensiblement cylindrique et présentant un diamètre inférieur au diamètre de la première partie sensiblement cylindrique, et une troisième partie sensiblement cylindrique présentant adjacente de la deuxième partie sensiblement cylindrique et présentant un diamètre inférieur au diamètre de la deuxième partie sensiblement cylindrique. Lesdites première et deuxième parties sensiblement cylindriques successives sont reliées entre elles par une zone intermédiaire formant un plateau ou marche s'étendant entre une partie sensiblement circulaire d'un bord de la première partie sensiblement cylindrique et une partie sensiblement circulaire d'un bord de la deuxième partie sensiblement cylindrique, lesdites parties sensiblement circulaires desdits bords desdites première et deuxième parties sensiblement cylindriques étant distantes l'une de l'autre d'une distance mesurée perpendiculairement à l'axe central de la cavité comprise entre 0,03 fois et 0,25 fois le diamètre de la première partie sensiblement cylindrique. Lesdites deuxième et troisième parties sensiblement cylindriques successives sont reliées entre elles par une zone intermédiaire formant un plateau ou marche s'étendant entre une partie sensiblement circulaire d'un bord de la deuxième partie sensiblement cylindrique et une partie sensiblement circulaire d'un bord de la troisième partie sensiblement cylindrique, lesdites parties sensiblement circulaires desdits bords desdites deuxième et troisième parties sensiblement cylindriques étant distantes l'une de l'autre d'une distance mesurée perpendiculairement à l'axe central de la cavité comprise entre 0,03 fois et 0,25 fois le diamètre de la deuxième partie sensiblement cylindrique.

Selon une forme de réalisation plus spécifiquement préférée, la cavité comporte au moins quatre parties sensiblement cylindriques successives à partir de l'ouverture, à savoir une première partie sensiblement cylindrique adjacente de l'ouverture présentant un premier diamètre, une deuxième partie sensiblement cylindrique adjacente de la première partie sensiblement cylindrique et présentant un diamètre inférieur au diamètre de la première partie sensiblement cylindrique, une troisième partie sensiblement cylindrique présentant adjacente de la deuxième partie sensiblement cylindrique et présentant un diamètre inférieur au diamètre de la deuxième partie sensiblement cylindrique, et une quatrième partie s'étendant entre le fond de la cavité, la quatrième partie étant avantageusement sensiblement cylindrique et présentant un diamètre inférieur au diamètre de la troisième partie sensiblement cylindrique. Lesdites première et deuxième parties sensiblement cylindriques successives sont reliées entre elles par une zone intermédiaire formant un plateau ou marche s'étendant entre une partie sensiblement circulaire d'un bord de la première partie sensiblement cylindrique et une partie sensiblement circulaire d'un bord de la deuxième partie sensiblement cylindrique, lesdites parties sensiblement circulaires desdits bords desdites première et deuxième parties sensiblement cylindriques étant distantes l'une de l'autre d'une distance mesurée perpendiculairement à l'axe central de la cavité comprise entre 0,03 fois et 0,25 fois le diamètre de la première partie sensiblement cylindrique. Lesdites deuxième et troisième parties sensiblement cylindriques successives sont reliées entre elles par une zone intermédiaire formant un plateau ou marche s'étendant entre une partie sensiblement circulaire d'un bord de la deuxième partie sensiblement cylindrique et une partie sensiblement circulaire d'un bord de la troisième partie sensiblement cylindrique, lesdites parties sensiblement circulaires desdits bords desdites deuxième et troisième parties sensiblement cylindriques étant distantes l'une de l'autre d'une distance mesurée perpendiculairement à l'axe central de la cavité comprise entre 0,03 fois et 0,25 fois le diamètre de la deuxième partie sensiblement cylindrique. Lesdites troisième et quatrième parties sensiblement cylindriques successives sont reliées entre elles par une zone intermédiaire formant un plateau ou marche s'étendant entre une partie sensiblement circulaire d'un bord de la troisième partie sensiblement cylindrique et une partie sensiblement circulaire d'un bord de la quatrième partie sensiblement cylindrique, lesdites parties sensiblement circulaires desdits bords desdites troisième et quatrième parties sensiblement cylindriques étant distantes l'une de l'autre d'une distance mesurée perpendiculairement à l'axe central de la cavité comprise entre 0,03 fois et 0,25 fois le diamètre de la troisième partie sensiblement cylindrique.

Selon une particularité avantageuse, le fond de la cavité est sensiblement bombée. De préférence le rayon de bombage du fond de la cavité est supérieur au rayon de bombage de la face extrême inférieure. L'épaisseur de la portion de la structure située entre le fond de la cavité et la face extrême est avantageusement d'au moins 1mm, de préférence comprise entre 2 et 5mm.

Selon un détail avantageux, la zone intermédiaire s'étendant entre deux parties sensiblement cylindriques successives de la cavité présente un profil courbe, avantageusement convexe.

Selon une caractéristique de formes de réalisation avantageuses, la cavité est associée à au moins une protubérance sensiblement parallèle à l'axe de la cavité, ladite protubérance s'étendant dans la cavité et vers son axe central et servant de moyen de guidage pour une extrémité d'un implant.

De préférence, la cavité est associée à deux ou trois protubérances s'étendant dans la cavité et servant de moyens de guidage pour le placement de l'extrémité d'un implant dans la cavité.

Avantageusement, la ou lesdites protubérances ne s'étendent pas sur toute la longueur de la cavité.

Par exemple, la ou lesdites protubérances ne s'étendent que le long d'une ou de parties de la cavité proches de l'ouverture.

Selon une particularité avantageuse, la ou les protubérances sont profilées de manière à présenter une forme plus effilée au voisinage de l'ouverture de la cavité ou à son extrémité tournée vers l'ouverture de la cavité.

Selon une particularité avantageuse, l'épaisseur de la structure au niveau de la partie adjacente de l'ouverture est plus faible que l'épaisseur de la structure au niveau du fond de la cavité. Une épaisseur plus importante au niveau du fond de la cavité ou de l'extrémité de la structure opposée à celle munie de l'ouverture permet la formation d'un volume osseux plus important, malgré le fait que cette portion ou extrémité soit éloignée de l'os ou de la structure osseuse sur lequel un implant doit être placé.

L'épaisseur de la structure varie ainsi avantageusement le long de sa longueur depuis l'ouverture vers l'extrémité en s'accroissant progressivement avec un ou plusieurs paliers, ceci assurant alors un effet de pompage de cellules osseuses vers la portion extrême de la structure dans laquelle s'étend le fond de la cavité.

Selon une forme de réalisation préférée, l'épaisseur moyenne (de la paroi latérale) de la structure au niveau de l'ouverture est supérieure à 0,2 mm, tandis que l'épaisseur moyenne (de la paroi latérale) de la structure au niveau du fond de la cavité est au moins égale à deux fois, avantageusement au moins trois l'épaisseur moyenne (de la paroi latérale) de la structure au niveau de l'ouverture.

Selon une particularité avantageuse, les parties sensiblement cylindriques ont une longueur variant entre 0,75 fois la longueur moyenne et 1,25 fois la longueur moyenne, la longueur moyenne des parties sensiblement cylindriques étant déterminée en divisant la longueur totale de la cavité par le nombre de parties. Avantageusement la partie de la cavité sensiblement cylindrique adjacente de l'ouverture a une longueur plus importante que celle de la partie de la cavité adjacente du fond de la cavité.

Selon une particularité d'une forme de réalisation préférée, la structure présente une face extérieure sensiblement cylindrique.

De préférence, la cavité de la structure est formée au moins partiellement par usinage mécanique, en particulier par forage. Selon une autre particularité avantageuse, la forme extérieure de la structure est au moins partiellement usinée mécaniquement.

Avantageusement, les granulats de biomatériau sont dispersés dans une phase de sulfate de calcium sensiblement de manière homogène. Les granulats sont de préférence relativement proches les uns des autres.

De préférence, la structure contient de l'eau, dont une partie est liée chimiquement au granulats de biomatériau, la teneur en eau chimiquement liée au biomatériau étant comprise entre 0,1% en poids et 1% en poids par rapport au poids des granulats de biomatériau sous forme sèche.

Selon une particularité avantageuse, les granulats de biomatériau présentent en tant que tels une densité apparente comprise entre 0,5 et 0,6 g/cm³.

Selon encore une autre particularité avantageuse, le sulfate de calcium se présente essentiellement sous forme dihydrate. Par exemple plus de 90% en poids (en particulier plus de 95% en poids, plus particulièrement plus de 98% en poids) du sulfate de calcium se présente sous forme dihydrate.

Avantageusement, la structure présente une porosité plus grande que celle d'une matrice de sulfate de calcium contenant la même quantité de biomatériau et préparée à pression atmosphérique à partir d'une pâte aqueuse de sulfate de calcium hémihydrate et de granulats de biomatériau et/ou un volume de bulles de gaz réduit par rapport au volume de bulles de gaz d'une matrice de sulfate de calcium préparée à pression atmosphérique à partir d'une pâte aqueuse de sulfate de calcium hemihydrate. Cette moindre porosité et/ou moindre volume de gaz présent dans la matrice de sulfate de calcium permet de ralentir la dissolution du sulfate de calcium et permet ainsi d'améliorer encore la croissance des tissus régénérant de l'os le long du revêtement ostéointégrable.

De préférence, la structure est constituée à plus de 90% en poids, avantageusement à plus de 95% en poids, de préférence à plus de 98% en poids (a) de sulfate de calcium sous forme dihydrate, (b) d'eau et/ou de sérum, et (c) de granulats de biomatériau.

Les pores de diamètre compris entre 50µm et 100µm du biomatériau sont avantageusement au moins partiellement remplis de sulfate de calcium. Par exemple au moins 10% (avantageusement au moins 15%, de préférence au moins 20%) du volume poreux formé par des pores de diamètre compris entre 50µm et 100µm des granulats de biomatériau phosphocalcique.

La structure est avantageusement placée, avant l'emploi, dans un emballage sous vide et stérile.

L'invention a encore pour objet un ensemble, en particulier kit, comprenant au moins une structure suivant l'invention (présentant une ou plusieurs caractéristiques de la structure de l'invention décrite ci avant dans le présent mémoire ou dans l'une quelconque des revendications), et un implant, avantageusement en titane, présentant un corps avec au moins un palier, ledit corps ayant une forme adaptée à la forme de la cavité de la structure.

L'invention a toujours pour objet une matière reconstituante d'os comprenant essentiellement du sulfate de calcium de qualité pharmaceutique et un biomatériau lentement ou non résorbable phosphocalcique ou apatite, ladite matière présentant une ou plusieurs caractéristiques de la matière reconstituante d'os d'une structure suivant l'invention telle que définie dans le présent mémoire ou dans l'une quelconque des revendications.

Toujours un objet de l'invention est un outil pour créer une perforation ou une cavité dans une structure osseuse, ledit outil comprenant:
- un insert en métal présentant une extrémité libre présentant un tranchant ou une pointe ou un angle ou inclinaison, et
- un dispositif adapté pour générer une cavitation sonique ou ultrasonique de l'insert et pour déplacer l'insert dans la structure osseuse,
ledit insert présentant un canal adapté pour amener un liquide sous pression à l'extrémité libre présentant le tranchant ou la pointe ou la face inclinée (extrême). De préférence, l'outil comporte un moyen assurant une étanchéité de l'os au voisinage de la face par laquelle l'insert est introduit et/ou assurant un contrôle de la pression du liquide s'écoulant dans le trou en formation.

Le système d'irrigation de cet outil permet de décoller la membrane de Schneider avant que l'outil ne transperce le sinus, ceci grâce à l'amenée d'un liquide sous pression lors de la cavitation de l'insert dans le tissu osseux.

L'invention a donc également pour objet une méthode de réalisation d'une cavité ou d'un trou dans un os ou une structure osseuse par cavitation sonique ou ultrasonique d'un insert avec une extrémité ou bord tranchant ou avec une extrémité avec une pointe. Ceci permet de lyser le tissu osseux. De plus, un tel traitement permet de détruire des bactéries et n'est pas traumatique pour les tissus environnants, de par l'absence de mouvement de rotation. Un meilleur contrôle de réchauffement de l'insert est également possible. Vu le moindre échauffement de l'os lors du traitement, on parvient à éviter une nécrose de l'os ou à limiter très fortement une telle nécrose si elle venait à se former.

L'invention a toujours pour objet une méthode de réalisation d'une cavité ou d'un trou dans un os ou une structure osseuse en contact avec une membrane, en particulier une membrane du sinus, dans laquelle on perce la cavité ou le trou dans l'os ou la structure osseuse au moyen d'un insert ou d'un foret tout en assurant l'injection d'un liquide sous pression par l'extrémité libre. La pression est avantageusement apte à faire traverser au moins une portion de l'os par le liquide.
Le passage de liquide à travers l'os ou partie d'os est amélioré ou mieux contrôlé en prévoyant un moyen d'étanchéité entre l'insert et l'os à transpercer.

Des particularités et détails de l'invention ressortiront de la description suivante
dans laquelle il est fait référence aux dessins ci-annexés.

Dans ces dessins,
- la figure 1 est une vue de côté d'un implant,

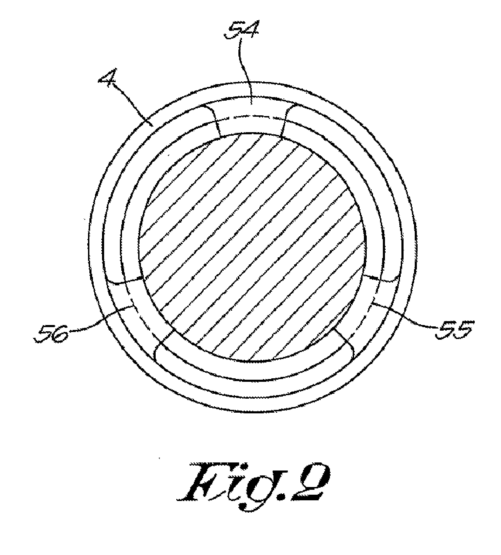

- la figure 2 est une vue en coupe de l'implant de la figure 1 le long de la ligne II-II:,

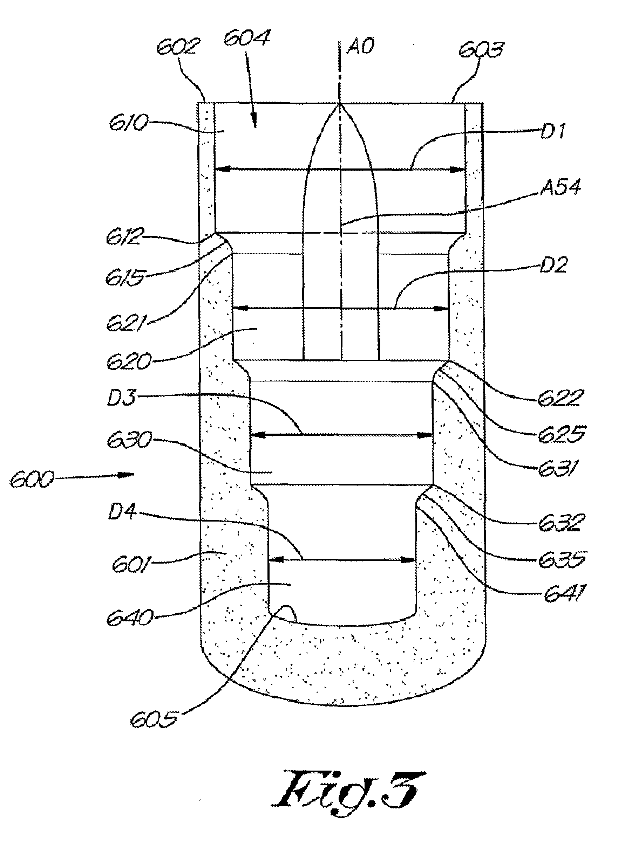

- La figure 3 est une vue en coupe d'une structure suivant l'invention adaptée pour recevoir une partie de l'implant de la figure 1 ;

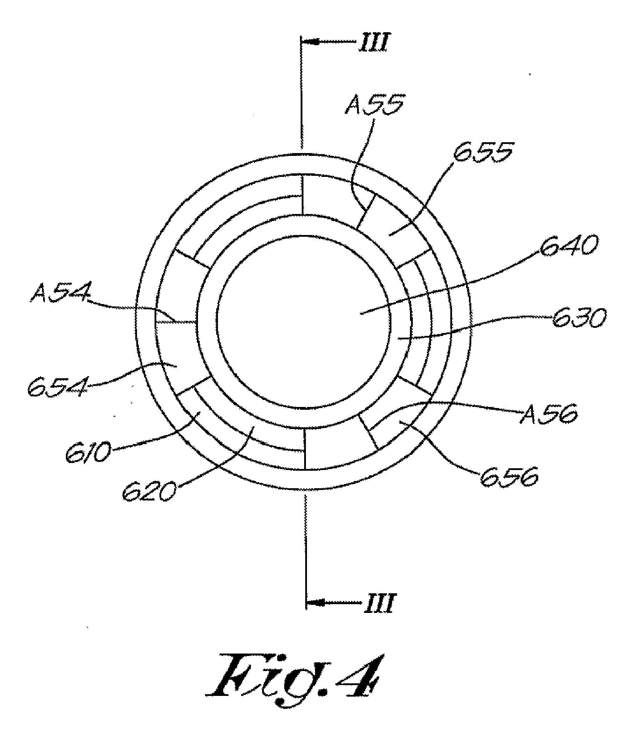

- La figure 4 est une vue de dessus (à partir de la face pourvue de l'ouverture) de la structure de la figure 3 ;
- la figure 5 est une vue de côté d'un outil pour former un trou par cavitation,
- la figure 6 est une vue de l'extrémité de l'outil de la figure 5,
- la figure 7 est une vue d'un autre outil pour former un trou dans le sinus,
- les figures 8A à 8D montrent schématiquement des étapes pour la mise en place d'un implant suivant l'invention,
- la figure 9 est une vue schématique d'un outil suivant l'invention,
- la figure 10 est une vue schématique (SEM) agrandie d'un agglomérat de biomatériau, et
- la figure 11 est une spectroscopie infrarouge (courbe absorbance a.u./longueur d'onde cm⁻¹) du biomatériau.

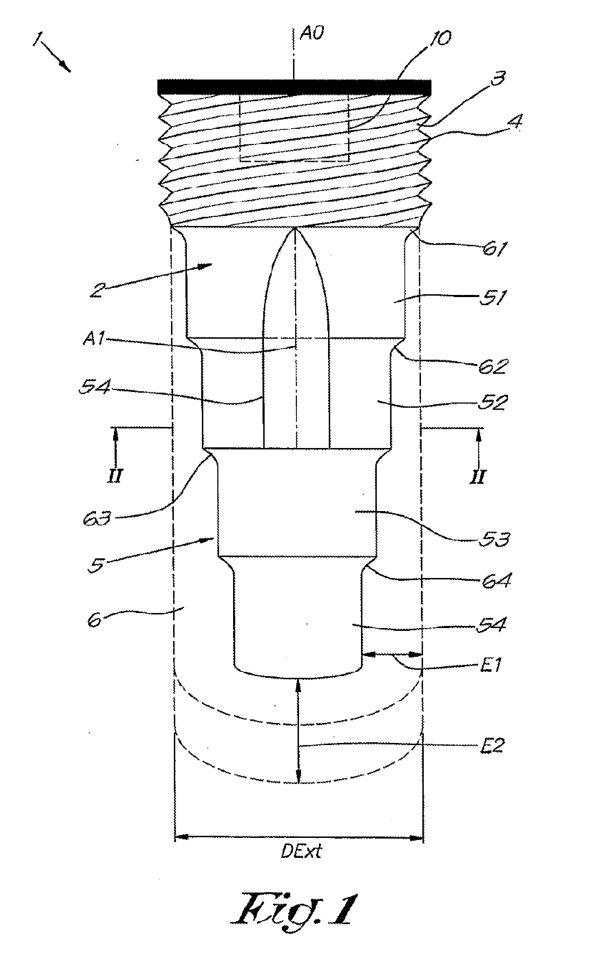

L'implant 1 de la figure 1 comprend un corps central 2 en titane dont une portion extrême 3 présente un filet 4 destiné à former une fixation primaire lors du placement au moins partiel de l'implant dans une cavité d'un os. Ce corps central 2 comporte une portion 5 constituée de quatre étages sensiblement cylindriques 51,52,53,54 disposés en pyramide les uns par rapport aux autres de manière à définir une série de paliers ou de plateau ou marche 61,62,63,64. L'étage 54 le plus éloigné de la portion filetée 3 a le diamètre le plus petit. Le diamètre des étages cylindriques diminue au plus que l'on s'éloigne de la portion filetée. Les deux étages 51,52 adjacents de la portion filetée 3 présentent trois canaux périphériques 70,71 ,72 dont l'axe A1 est parallèle à l'axe central A0 de l'implant. La différence entre le diamètre d'un premier étage et le diamètre d'un étage directement adjacent dudit premier étage est sensiblement constant.

La portion 5 est destinée à être insérée dans une cavité d'une structure ostéointégrable 6 dont le diamètre extérieur DExt est sensiblement constant et sensiblement égal au diamètre du creux du filet 4 de la portion 3. L'extrémité du revêtement 6 est sensiblement bombée.

La structure 6 est préparée en mélangeant du sulfate de calcium dihydrate avec des granulats de particules d'Algipore® (Friadent GmbH) inférieure à 500µm, lesdites granulats poreux phosphocalciques présentant en tant que telles une surface spécifique d'environ 15m²/g, une macroporosité constituée de pores ou d'alvéoles de diamètre moyen compris entre 50 µm et 100 µm. La densité apparente des particules poreuses était d'environ 0,55 g/ml.

Le sulfate de calcium dihydrate de qualité pharmaceutique et présentant sous forme sèche une teneur en magnésium de moins de 2% en poids (avantageusement de moins de 1% en poids) a été mélangé à de l'eau pour former une pâte. A cette pâte, on a ajouté la fraction granulométrique inférieure à 500µm des particules phosphocalciques.

Cette opération de mélange a été effectuée sous pression réduite, avantageusement sous vide ou sous un certain vide.

La pression était inférieure à la pression atmosphérique, par exemple inférieure à 0,5 10⁵ Pa (0,5 bar), mais avantageusement encore moins (moins de 0,1 10⁵ Pa, en particulier moins de 0,03 10⁵ Pa) lors de l'opération de mélange des ingrédients.
Ceci permet de réduire la formation de bulles dans la matrice de sulfate de calcium.

De façon avantageuse, les particules poreuses phosphocalciques sont traitées sous vide avant de les incorporer au mélange sulfate de calcium + eau.

On a remarqué qu'en opérant à faible pression, en particulier sous vide, il était possible de réduire la porosité du sulfate de calcium dihydrate. On a également remarqué que des pores de particules poreuses phosphocalciques contenaient du sulfate de calcium.

La structure 6 est décrite ci-après.

Comme on le voit la structure 6 est indépendante de l'implant avant l'ostéointégration. Ceci permet un placement plus aisé des différents éléments après contrôle de qualité de ceux-ci. Enfin, la formation d'os entre la structure et l'implant permettra encore une meilleure fixation de l'implant dans un volume osseux plus important, par formation d'os entre la structure et l'implant,

La structure 6 a la forme d'un manchon 600 dont une extrémité est obturée. La structure comprend un corps ostéogène 601 en une matière reconstituante d'os comprenant essentiellement du sulfate de calcium de qualité pharmaceutique et un biomatériau lentement ou non résorbable phosphocalcique ou apatite sous forme de granulats poreux présentant une granulométrie moyenne en poids comprise entre 250 et 3000µm, en particulier entre 300µm et 1000µm. Lesdits granulats de biomatériau présentant en tant que telles une surface spécifique comprise entre 10 et 50m²/g (en particulier entre 10m²/g et 20m²/g), une macroporosité formée de pores de diamètre moyen (en volume poreux) compris entre 50 et 100 µm. Ledit corps ostéogène présentant une extrémité ou face 602 sensiblement plane avec une ouverture 603 prolongée par une cavité centrale 604 avec un axe central A0 et adaptée pour recevoir au moins une extrémité d'un implant du type représenté à la figure 1.

La cavité 604 comporte au moins quatre parties sensiblement cylindriques successives à partir de l'ouverture, à savoir une première partie 610 sensiblement cylindrique adjacente de l'ouverture 603 présentant un premier diamètre D1, une deuxième partie sensiblement cylindrique 620 adjacente de la première partie sensiblement cylindrique 610 et présentant un diamètre D2 inférieur au diamètre D1 de la première partie sensiblement cylindrique, une troisième partie sensiblement cylindrique 630 présentant adjacente de la deuxième partie sensiblement cylindrique 620 et présentant un diamètre D3 inférieur au diamètre D2 de la deuxième partie sensiblement cylindrique, et une quatrième partie 640 s'étendant entre le fond 605 de la cavité 604 et la troisième partie 630, la quatrième partie 640 étant avantageusement sensiblement cylindrique et présentant un diamètre D4 inférieur au diamètre D3 de la troisième partie sensiblement cylindrique.

Lesdites première et deuxième parties 610,620 sensiblement cylindriques successives sont reliées entre elles par une zone intermédiaire 615 formant un plateau ou marche ou un palier s'étendant entre une partie sensiblement circulaire d'un bord 612 de la première partie sensiblement cylindrique 610 et une partie sensiblement circulaire d'un bord 621 de la deuxième partie sensiblement cylindrique 620. Lesdites parties sensiblement circulaires desdits bords 612, 621 desdites première et deuxième parties 610,620 sensiblement cylindriques sont distantes l'une de l'autre d'une distance (distance égale à D1 - D2 divisé par deux) mesurée perpendiculairement à l'axe central A0 de la cavité 604 comprise entre 0,03 fois et 0,25 fois le diamètre D1 de la première partie sensiblement cylindrique 610. Lesdites deuxième et troisième parties 620,630 sensiblement cylindriques successives sont reliées entre elles par une zone intermédiaire 625 formant un plateau ou marche ou pallier s'étendant entre une partie sensiblement circulaire d'un bord 622 de la deuxième partie 620 sensiblement cylindrique et une partie sensiblement circulaire d'un bord 631 de la troisième partie 630 sensiblement cylindrique. Lesdites parties sensiblement circulaires desdits bords 622, 631 desdites deuxième et troisième parties 620, 630 sensiblement cylindriques sont distantes l'une de l'autre d'une distance (distance égale à D2 - D3 divisé par 2) mesurée perpendiculairement à l'axe central A0 de la cavité 604 comprise entre 0,03 fois et 0,25 fois le diamètre D2 de la deuxième partie sensiblement cylindrique 620. Lesdites troisième et quatrième parties sensiblement cylindriques successives 630,640 sont reliées entre elles par une zone intermédiaire 635 formant un plateau ou marche ou un pallier s'étendant entre une partie sensiblement circulaire d'un bord 632 de la troisième partie 630 sensiblement cylindrique et une partie sensiblement circulaire d'un bord 641 de la quatrième partie 640 sensiblement cylindrique, lesdites parties sensiblement circulaires desdits bords 632.641 desdites troisième et quatrième parties 630, 640 sensiblement cylindriques étant distantes l'une de l'autre d'une distance (distance égale à D3 - D4 divisé par 2) mesurée perpendiculairement à l'axe central A0 de la cavité 604 comprise entre 0,03 fois et 0,25 fois le diamètre D3 de la troisième partie sensiblement cylindrique 630.

Le fond 605 de la cavité 604 est courbe (forme sensiblement bombée), le point central du fond étant le plus éloigné de l'ouverture 603.

Les zones intermédiaires 615, 625, 635 s'étendant entre deux parties sensiblement cylindriques successives de la cavité 604 présentent un profil courbe, avantageusement convexe. Ainsi la face de ces zones intermédiaires se trouve tournée vers la cavité par rapport à une ligne s'étendant entre un point d'une partie sensiblement circulaire d'un bord d'une première partie sensiblement cylindrique et entre un autre point d'une partie sensiblement circulaire d'un bord d'une autre partie sensiblement cylindrique adjacente ou successive à la première.

La cavité 604 est associée à trois protubérances sensiblement parallèles à l'axe A0 de la cavité 604, lesdites protubérances s'étendant dans la cavité et vers son axe central et servant de moyen de guidage pour une extrémité d'un implant.

La cavité 604 est associée à deux ou trois protubérances s'étendant dans la cavité et servant de moyens de guidage pour le placement de l'extrémité d'un implant (voir figure 1) dans la cavité 604. Ces protubérances 654,655,656 présentent chacune un profilé effilé avec un plan de symétrie. Les plans de symétrie A54, A55, A56 sont disposés à 120° l'un de l'autre par rapport à l'axe A0, lesdits plans se coupant le long de l'axe A0.

L'extrémité des protubérances 654,655,656 tournée vers l'ouverture 603 forme une pointe ou une partie de faible largeur pour favoriser le glissement des canaux 70, 71, 72, et le positionnement de l'implant 1 par rapport à la cavité. Le bord libre d'un ou de canaux 70,71,72 est apte à glisser sur la paroi latérale profilé d'une protubérance.

Lorsque la structure présente de telles protubérances 654, 655, 656, la structure et l'implant sont mis ensemble avant de fixer l'implant avec la portion filetée dans de l'os ou une structure osseuse.

L'épaisseur de la structure au niveau de la partie adjacente de l'ouverture 603 est plus faible que l'épaisseur de la structure au niveau du fond 605 de la cavité 604.
Cette épaisseur s'accroît avec des sauts d'épaisseur.

L'épaisseur moyenne de la structure au niveau de l'ouverture 603 est supérieure à 0,2 mm, tandis que l'épaisseur moyenne de la structure au niveau du fond 605 de la cavité est au moins égale à deux fois, avantageusement au moins trois l'épaisseur moyenne de la structure au niveau de l'ouverture.

Les parties sensiblement cylindriques 610,620,630,640 ont une longueur L sensiblement égale.

La structure est avantageusement préparée à partir d'un bloc réalisé en matière formant de l'os. Ce bloc est alors usiné pour former une cavité en plusieurs parties, et pour façonner la paroi extérieure de la structure. Ceci permet de donner à la structure des faces présentant la porosité ou des pores correspondant aux pores intérieurs du bloc avant son façonnage. Cet usinage est par exemple, un usinage mécanique, en particulier par forage.

La structure 6 comporte avantageusement des granulats de biomatériau dispersés dans une phase de sulfate de calcium sensiblement de manière homogène. Elle contient de l'eau, dont une partie est liée chimiquement aux granulats de biomatériau, la teneur en eau (de constitution) chimiquement liée au biomatériau étant comprise entre 0,1% en poids et 1% en poids par rapport au poids des granulats de biomatériau.

Les granulats de biomatériau présentent en tant que tels une densité apparente comprise entre 0,5 et 0,6 g/cm³.

Le sulfate de calcium se présente essentiellement sous forme dihydrate.

La structure présente une porosité plus grande que celle d'une matrice de sulfate de calcium contenant la même quantité de biomatériau et préparée à pression atmosphérique à partir d'une pâte aqueuse de sulfate de calcium hémihydrate et de granulats de biomatériau.

En particulier, la structure est constituée à plus de 90% en poids, avantageusement à plus de 95% en poids, de préférence à plus de 98% en poids de sulfate de calcium sous forme dihydrate, d'eau et/ou de sérum, et de granulats de biomatériau.

Différents mélanges ont ainsi été préparés, le rapport en poids sulfate de calcium dihydrate/particules phosphocalciques des mélanges étant les suivants :
Mélange 1 : rapport en poids sous forme sèche
   sulfate de calcium dihydrate/granulats phosphocalciques de 3
Mélange 2 : rapport en poids sous forme sèche
   sulfate de calcium dihydrate/granulats phosphocalciques de 2,5
Mélange 3 : rapport en poids sous forme sèche
   sulfate de calcium dihydrate/granulats phosphocalciques de 2
Mélange 4 : rapport en poids sous forme sèche
   sulfate de calcium dihydrate/granulats phosphocalciques de 1,5
Mélange 5 : rapport en poids sous forme sèche
   sulfate de calcium dihydrate/granulats phosphocalciques de 1

Les mélanges avec un rapport en poids sous forme sèche sulfate de calcium dihydrate/granulats poreux phosphocalciques compris entre 1 et 2,5 sont préférés. Le mélange est avantageusement préparé sous vide (vide partiel ou à pression réduite) et est versé sous vide dans un moule. Le mélange peut contenir un ou des autres agents, tensioactifs, polymères, charges, etc, la teneur en autres agents, additifs et charges étant avantageusement de moins de 10% en poids de la structure sèche (séché à 80°C pour éliminer toute l'eau libre), en particulier de moins de 5% en poids de la structure. De tels agents sont par exemple des agents polysaccharide apte à former un gel, l'acide hyaluronique, des antibiotiques, des facteurs de croissance, etc.

La portion filetée 3 de l'implant 1 présente un système d'attache 10 par exemple pour une dent, une prothèse, une articulation, etc. Ce système d'attache 10 est par exemple un évidement avec un filet réalisé dans la tête filetée 3 pour la fixation d'une dent, d'une prothèse, d'une articulation...

Pour le placement de l'implant dans le maxillaire supérieur, il est avantageux de conserver l'intégrité de la membrane du sinus.

Pour ce faire on utilise un outil permettant à la fois de perforer la structure osseuse, tout en assurant lorsque l'outil ou l'insert est proche de la membrane (tout en étant encore dans l'os) d'assurer un décollement progressif de la membrane du sinus.

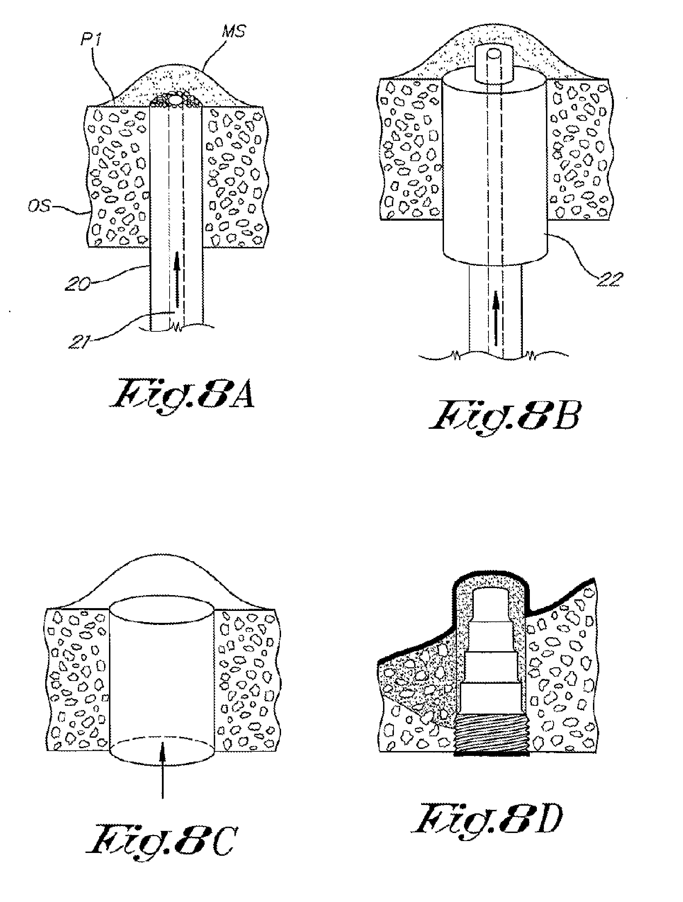

Dans un premier procédé (voir figures 8), on fore la structure osseuse (OS) avec un foret de guidage 20 présentant un canal central 21 pour amener un liquide sous pression. L'os étant une structure poreuse, le liquide sous pression du canal s'écoule partiellement dans l'os. Toutefois, tant que l'extrémité libre du foret de guidage est éloignée de la paroi osseuse interne P1, la pression du liquide dans le canal est sensiblement constante. Lorsque l'extrémité du foret est adjacente de la paroi interne P1, le liquide sous pression s'écoule facilement dans l'os vers la membrane du sinus MS, ce qui permet d'assurer un décollement progressif de la membrane avant que le foret de guidage 20 ne transperce la structure osseuse OS.

La mise sous pression du liquide peut être continue ou non, par exemple par étape successive. Par exemple, le liquide est mis sous un cycle de pressurisation comportant des étapes de haute pression et des étapes de basse ou moyenne pression et/ou d'absence de pression.

Cette chute de pression permet également d'avertir le praticien que la structure osseuse OS va bientôt être transpercée. Ce signal est également avantageusement utilisé pour commander une injection ou le contrôle d'une injection d'un volume de liquide précis pour contrôler un décollement adéquat de la membrane du sinus. Par exemple, une fois le signal de chute de pression détecté, un volume de 5ml de liquide aseptique (eau) est injecté. Lorsque les 5 ml de liquide sont injectés, la rotation du foret de guidage est interrompue.

Pour assurer que le liquide sous pression passe bien au travers de l'os, il est avantageux que l'outil soit muni d'un moyen assurant une étanchéité de l'os au voisinage de la face par laquelle l'insert est introduit et/ou assurant un contrôle de la pression du liquide s'écoulant dans le trou en formation (par exemple pour le refroidissement de l'insert et des parois intérieures du trou lors de son percement).

On fore ensuite le long du foret de guidage un trou de diamètre supérieur au moyen d'un ou de plusieurs forets 22. Lors du forage complémentaire le long du foret de guidage pour élargir le diamètre du trou, il peut être opportun de contrôler la pression de liquide du canal 21 du foret de guidage. Par exemple, dès que cette pression tombe sous un niveau déterminé, un dispositif de contrôle amène à nouveau de l'eau sous pression de manière à assurer que la membrane du sinus sera toujours décollée correctement et éloignée de la structure osseuse au voisinage de l'endroit du trou à forer.

Lorsque le trou a un diamètre correspondant sensiblement au diamètre de la portion filetée de l'implant on retire le foret et le foret de guidage.

On injecte alors par le trou une composition de greffage et on insère ensuite l'implant. Grâce au filet de la portion 3, il est possible d'avoir une stabilisation primaire de l'implant sur la structure osseuse, avant que la stabilisation secondaire soit réalisée grâce à la transformation du revêtement en une couche osseuse par une colonisation rapide du revêtement par des cellules osseuses du patient.

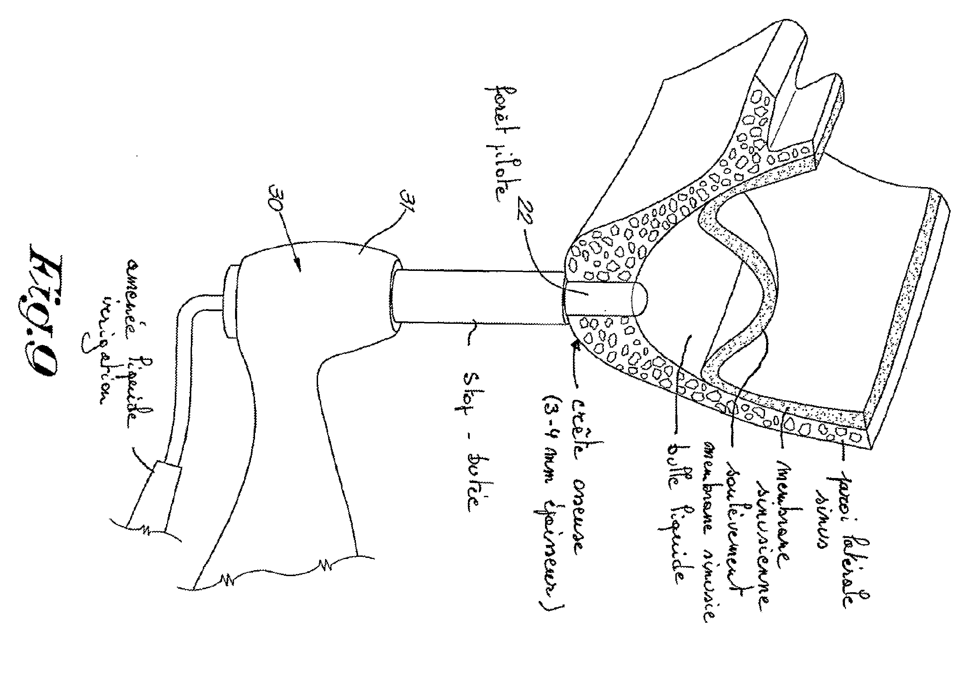

La figure 9 est une vue schématique en perspective d'un appareil de perforation portant un foret 22.

Cet appareil comporte un support 30 dont la tête 31 est munie de moyen pour entraîner en rotation le foret 22. La tête 31 est associée à une butée 32 dont la position est avantageusement adaptable en fonction de la longueur du mouvement de percement de l'os avec le foret de guidage, longueur définie par une longueur correspondant sensiblement à la longueur pour laquelle une chute de pression est déterminée.

Au lieu d'utiliser un ou des forets pour transpercer la structure osseuse ou pour former un trou destiné à recevoir l'implant, on a utilisé un outil comprenant un insert 40 en métal présentant une ou des parties tranchantes 41 avec une tête extrême recouverte de diamants 42 et un dispositif générant une cavitation ultrasonique ou sonique (en particulier ultrasonique) de l'insert dans la structure osseuse tout en assurant un mouvement d'avancement de l'insert dans la structure osseuse.

L'insert présente un canal intérieur 43 adapté pour amener un liquide sous pression.

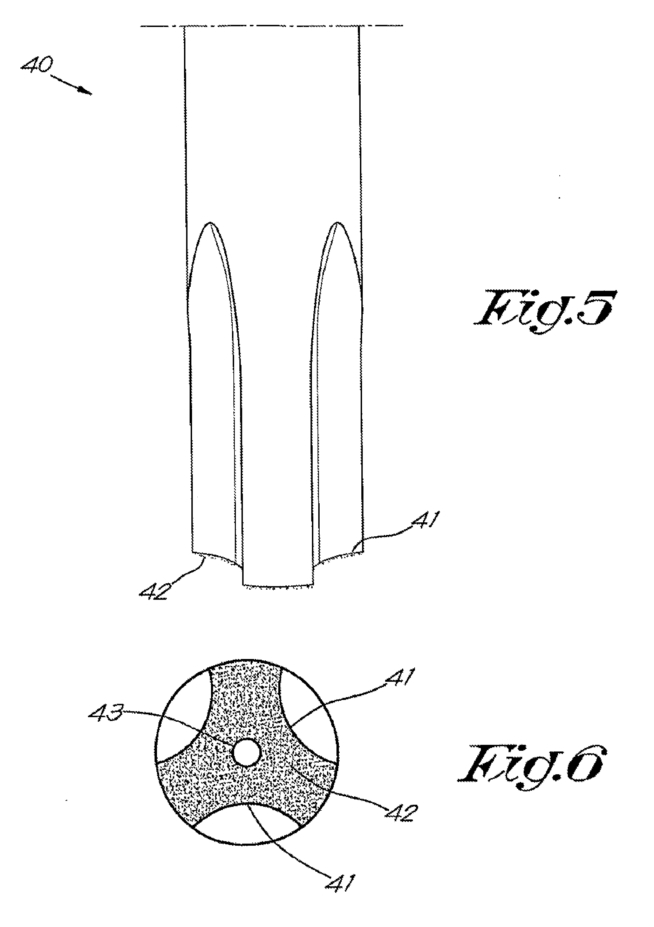

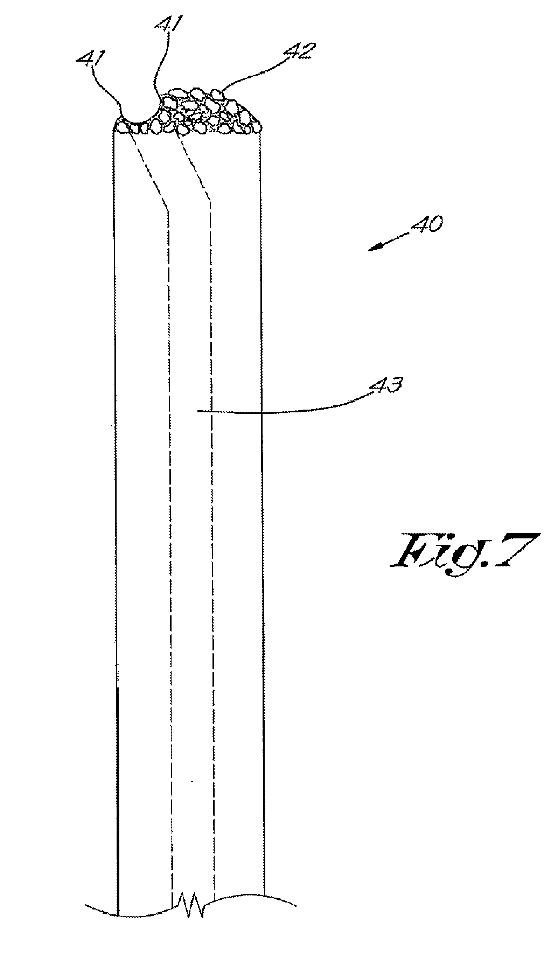

La figure 7 est une vue d'un autre insert similaire à celui de la figure 5. La longueur totale de l'insert 40 est par exemple de moins de 35mm.

Pour assurer que le liquide sous pression passe bien au travers de l'os, il est avantageux que l'outil soit muni d'un moyen assurant une étanchéité de l'os au voisinage de la face par laquelle l'insert est introduit et/ou assurant un contrôle de la pression du liquide s'écoulant dans le trou en formation (par exemple pour le refroidissement de l'insert et des parois intérieures du trou lors de son percement).

L'insert présente une élasticité dans le sens et à contresens de la progression de l'insert dans l'os. L'insert va être mis en vibration dans le sens de la progression de l'insert dans l'os en créant des sortes de bulles de cavitation.

Lors de l'opération de percement d'un trou par cavitation ultrasonique, un liquide est amené sous pression par le canal intérieur de l'insert. Ce liquide est par exemple de l'eau, du sérum physiologique, une solution isotonique, une solution d'eau contenant 0,5% en poids à 3% en poids de NaCl, par exemple de 0,7 à 1,5% en poids de NaCl. Ce liquide est mis sous pression de manière continue ou non.

Lorsque le liquide est mis sous pression, une partie de ce liquide passe dans la structure poreuse de l'os en se frayant des chemins de passage au travers de la masse osseuse du type II, III et IV. Tant que l'extrémité libre de l'insert est trop éloignée de la membrane du sinus, la pression du liquide dans le canal est importante.

Une fois que l'extrémité de l'insert est adjacente de la paroi osseuse en contact avec la membrane de Schneider (membrane du sinus), le liquide va passer au travers de l'os pour décoller la membrane de Schneider. Une poche de liquide va alors se former entre la bas fond sinusien et la membrane.

On a remarqué que la création d'un chenal osseux par cavitation ultrasonique permettait de lyser le tissu osseux, par exemple de type II, III et IV. Une telle lyse induite mécaniquement est utile chirurgicalement pour créer un chenal dans l'os de manière non traumatique pour les tissus environnants, mais en détruisant les bactéries.

L'échauffement de l'insert est mieux contrôlable par exemple au moyen d'un liquide d'irrigation, ce qui permet d'éviter des nécroses au niveau du trou, mais surtout ce qui s'est avéré utile pour assurer une meilleure ostéointégration, en particulier en utilisant l'implant selon l'invention.

En utilisant un chenal créé par cavitation ultrasonique et un implant selon l'invention (implant portant une rotule ou présentant un dispositif d'accrochage pour une rotule), il est possible d'obtenir une ostéointégration des prothèses orthopédiques. L'implant selon l'invention est symbiotique, l'ostéointégration étant ostéosymbiotique.
Dans le cas d'un os de type I, par exemple des fûts tumoraux, on a remarqué que l'implant suivant l'invention grâce à son revêtement particulier permet de compenser l'inertie de la biodynamique ostéogénérative, et permet une ostéointégration de l'implant othopédique. Ceci permettra d'apporter une stabilité totale de l'implant après ostéointégration. Pour assurer une stabilisation primaire, l'implant est fixé à l'os au moyen du filet de la partie supérieure de l'implant. Après cette stabilité primaire, la création de tissu osseux sur les faces de la structure et dans la structure va former une gangue osseuse autour du corps en titane qui assurera alors une stabilité secondaire.

L'implant peut comprendre directement un plateau ou marche articulaire, une tête fémorale, etc., mais est avantageusement du type présentant un moyen permettant, avantageusement après ostéointégration de l'implant, le placement et la fixation d'un plateau ou marche, d'une articulation, d'une tête fémorale, etc. La structure suivant l'invention peut donc être utilisée dans les os du coude, de l'épaule, du genou, de la hanche, c'est-à-dire tous les os longs en général.

La structure suivant l'invention, et donc la matière reconstituante d'os s'est avéré présenter un effet catalytique pour la formation d'os, en particulier dans des os à faible propension à l'ostéointégration.

Un biomatériau particulièrement utile pour la structure suivant l'invention a la forme de granulats poreux, dont la composition correspond essentiellement à de l'hydroxyapatite carbonate avec des traces de matières organiques du type protéine. La teneur en eau de constitution est de l'ordre de 0,2 à 1% en poids, la teneur en matière organique est comprise entre 2% en poids et 3% en poids, tandis que la phase minérale constitue le reste. La teneur en carbonate de calcium est de l'ordre de 2 à 3% en poids.

La figure 9 donne le graphe de diffraction aux rayons X du biomatériau préféré, mesuré ou converti à la longueur d'onde de 1,54 Â. Comme il ressort de ce graphe, l'intensité est faible démontrant une phase cristalline modérée hydroxyapatite. Cette courbe de diffraction RX est très similaire de celle d'une poudre d'os stérilisé d'origine humaine.

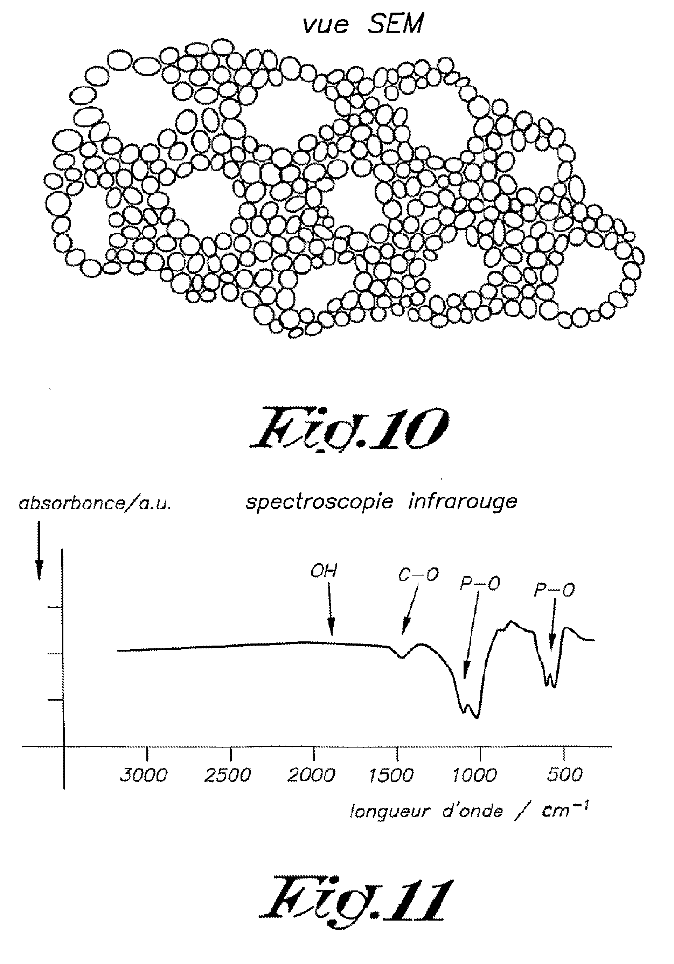

La figure 10 est une représentation schématique SEM d'un biomatériau préféré.

## Revendications

1. Outil pour créer une perforation ou une cavité dans un os ou une structure osseuse en contact avec une membrane, ledit outil comprenant:
- un foret (20, 22) présentant une extrémité libre et un canal (21) adapté pour amener un liquide sous pression à l'extrémité libre;
- un moyen pour fournir le liquide sous pression de telle sorte que le liquide s'écoule partiellement dans et à travers de l'os ou de la structure osseuse vers la membrane en perçant l'os ou la structure osseuse avec le foret, permettant d'assurer un décollement progressif de la membrane lorsque le foret est proche de la membrane tout en étant encore dans l'os ou la structure osseuse.

2. Outil suivant la revendication précédente, **caractérisée en ce que** la membrane est une membrane du sinus (MS).

3. Outil suivant l'une quelconque des revendications précédentes, **caractérisée en ce que** l'os est un maxillaire supérieur.

4. Outil suivant l'une quelconque des revendications précédentes comprenant en outre un moyen pour détecter une chute de pression du liquide.

5. Outil suivant la revendication précédente comprenant en outre un moyen pour commander une injection ou contrôler une injection d'un volume de liquide précis pour contrôler un décollement adéquat de la membrane.

6. Outil suivant l'une quelconque des revendications précédentes comprenant en outre un moyen pour mettre le liquide sous pression de manière continue ou discontinue.

7. Outil suivant l'une quelconque des revendications précédentes comprenant en outre un moyen d'étanchéité entre le foret et l'os ou la structure osseuse à transpercer.

8. Outil suivant l'une quelconque des revendications précédentes comprenant en outre une butée (32).

9. Procédé pour créer une perforation ou une cavité dans un os ou une structure osseuse en contact avec une membrane, le procédé comprenant les étapes de :
- percer l'os ou la structure osseuse avec un foret (20, 22);
- amener un liquide sous pression à une extrémité libre et par un canal (21) du foret de telle sorte que le liquide s'écoule partiellement dans et à travers de l'os ou de la structure osseuse vers la membrane en perçant l'os ou la structure osseuse avec le foret, permettant d'assurer un décollement progressif de la membrane lorsque le foret est proche de la membrane tout en étant encore dans l'os ou la structure osseuse.

10. Procédé suivant la revendication 9, **caractérisée en ce que** la membrane est une membrane du sinus (MS).

11. Procédé suivant la revendication 9 ou 10, **caractérisée en ce que** l'os est un maxillaire supérieur.

12. Procédé suivant l'une quelconque des revendications 9 à 11 comprenant en outre l'étape de détecter une chute de pression du liquide.

13. Procédé suivant la revendication 12 comprenant en outre l'étape de commander une injection ou contrôler une injection d'un volume de liquide précis pour contrôler un décollement adéquat de la membrane.

14. Procédé suivant la revendication 13 comprenant en outre l'étape d'interrompre la rotation du foret (20, 22) lors de l'injection du volume de liquide précis.

15. Procédé suivant l'une quelconque des revendications 9 à 14 comprenant en outre l'étape de mettre le liquide sous pression de manière continue ou discontinue.
